# EUROPEAN PATENT APPLICATION

(11) **EP 4 840 867 A1**
(43) Date of publication of application: **18.11.2026**
(21) Application number: 25738712.6
(22) Date of filing: 15.01.2025
(51) Int. Cl.: C07F 9/46, C07F 11/00, C07C 2/32, C07C 11/02, C07C 11/107, B01J 31/18, B01J 31/16

(54) **LIGAND COMPOUND, ORGANIC CHROMIUM COMPOUND, AND CATALYST COMPOSITION COMPRISING SAME**

(30) Priority: 11.01.2024 KR 20240005035
(71) Applicant: LG Chem, Ltd., Seoul 07336 (KR)
(72) Inventor: KIM, Seok Sun, Daejeon 34122 (KR); CHO, Yeon Ho, Daejeon 34122 (KR); LIM, Won Taeck, Daejeon 34122 (KR); JUNG, Seung Hwan, Daejeon 34122 (KR); SHIN, Eun Ji, Daejeon 34122 (KR); KIM, Tae Hee, Daejeon 34122 (KR)
(74) Representative: Goddar, Heinz J.
(86) International application number: PCT/IB2025/050419
(87) International publication number: WO 2025/150000

(57) **Abstract**

The present invention relates to a ligand compound having a novel structure, an organochromium compound, a catalyst composition comprising the organochromium compound which shows high catalyst activity and high selectivity to 1-hexene and 1-octene, and may perform ethylene oligomerization with excellent efficiency, and a method for oligomerizing ethylene using the same.

## Description

### TECHNICAL FIELD

### [Cross-reference to Related Applications]

This application claims the benefit of Korean Patent Application No. 10-2024-0005035, filed on January 11, 2024, in the Korean Intellectual Property Office, the contents of which are incorporated herein by reference.

### [Technical Field]

The present invention relates to a ligand compound, an organochromium compound, a catalyst composition comprising the organochromium compound, and a method for oligomerizing ethylene using the same.

### BACKGROUND ART

Linear alpha-olefins like 1-hexene and 1-octene are used as detergents, lubricants, plasticizers or the like, and particularly, are mainly used as comonomers for adjusting the density of polymers during preparing linear low-density polyethylene (LLDPE).

In the preparation process of the conventional linear low-density polyethylene (LLDPE), the copolymerization of ethylene together with comonomers like alpha-olefins such as 1-hexene and 1-octene has been performed in order to adjust the density by forming a branch in a polymer backbone.

Accordingly, in order to prepare the LLDPE with the high comonomer content, there were defects of the cost of the comonomer accounted for a big part of the manufacturing cost. To solve such defects, various attempts have been conducted.

The linear alpha-olefins were mainly produced through a Shell Higher Olefin Process. However, according to the method, alpha-olefins with various lengths were synthesized simultaneously according to Schultz-Flory distribution, and a separate separation process was inconveniently required for obtaining a specific alpha-olefin.

To solve such defects, a method of selectively synthesizing 1-hexene through the trimerization reaction of ethylene, or selectively synthesizing 1-octene through the tetramerization reaction of ethylene has been suggested. In addition, more studies on a catalyst system enabling such selective oligomerization of ethylene have been conducted.

### [Prior Art Document]

### [Patent Document]

(Patent Document 1) US 5064802 B2

### DETAILED DESCRIPTION OF THE INVENTION

### TECHNICAL PROBLEM

A task to solve in the present invention is to provide a ligand compound having a novel structure, an organochromium compound and a catalyst composition comprising the same which shows high catalyst activity and high selectivity to 1-hexene and 1-octene, and at the same time, significantly reduces the production of the isomers of 1-hexene and 1-octene, thereby enabling ethylene oligomerization with excellent efficiency.

### TECHNICAL SOLUTION

To solve the above task, the present invention provides a ligand compound, an organochromium compound, a catalyst composition, and a method for oligomerizing ethylene.
(1) The present invention provides a ligand compound represented by Formula 1.

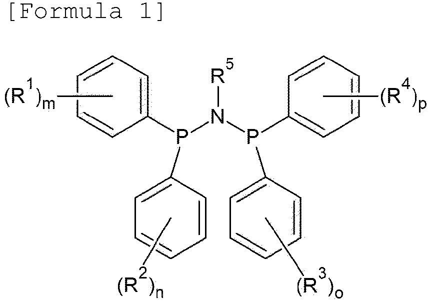

   In Formula 1, R¹ to R⁴ are each independently an alkyl group of 1 to 20 carbon atoms, a cycloalkyl group of 5 to 20 carbon atoms, an alkoxy group of 1 to 20 carbon atoms, or - Si(R⁷)₃, m, n, o, and p are each independently 0 or an integer selected from 1 to 5, where if m, n, o, and p are 2 or more, a plurality of R¹ to R⁴ are the same or different from each other, R⁵ is an alkyl group of 21 to 30 carbon atoms, and R⁷ is an alkyl group of 1 to 20 carbon atoms, where a plurality of R⁷ are the same or different from each other.
(2) The present invention provides the ligand compound according to (1), wherein R¹ to R⁴ are each independently an alkyl group of 1 to 20 carbon atoms, or -Si(R⁷)₃.
(3) The present invention provides the ligand compound according to (1) or (2), wherein R¹ to R⁴ are each independently -Si(R⁷)₃, and R⁷ is an alkyl group of 1 to 10 carbon atoms, where a plurality of R⁷ are the same or different from each other.
(4) The present invention provides the ligand compound according to any one among (1) to (3), wherein R¹ to R⁴ are each independently a trimethylsilyl group, a triethylsilyl group, a tri-n-propylsilyl group, a tri-n-butylsilyl group, a tri-n-octylsilyl group or a dimethyl(n-octyl)silyl group.
(5) The present invention provides the ligand compound according to any one among (1) to (4), wherein m, n, o and p are 1.
(6) The present invention provides the ligand compound according to any one among (1) to (5), wherein R⁵ is a branched alkyl group of 21 to 30 carbon atoms.
(7) The present invention provides the ligand compound according to any one among (1) to (6), wherein the ligand compound represented by Formula 1 is represented by Formula 2 or Formula 3 below. 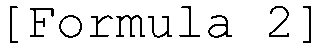 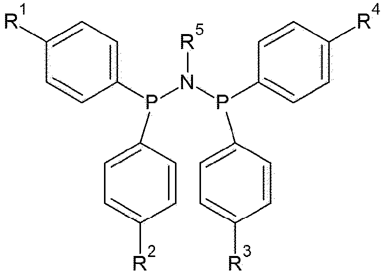 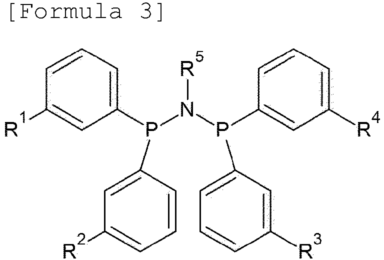 In Formula 2 and Formula 3, R¹ to R⁵ are the same as defined in (1) to (6).
(8) The present invention provides the ligand compound according to any one among (1) to (6), wherein the ligand compound represented by Formula 1 is represented by Formula 4 below.

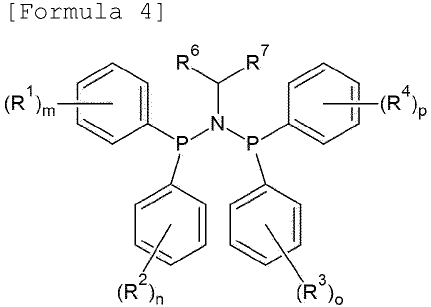

   In Formula 4, R¹ to R⁴, m, n, o and p are the same as defined in (1) to (6), and R⁶ and R⁷ are each independently an alkyl group of 1 to 27 carbon atoms, where the sum of carbon atoms of R⁶ and R⁷ is 20 to 29.
(9) The present invention provides the ligand compound according to any one among (1) to (8), wherein the ligand compound represented by Formula 1 is represented by Formula 5 or Formula 6 below.

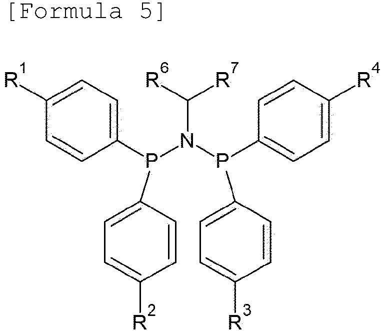

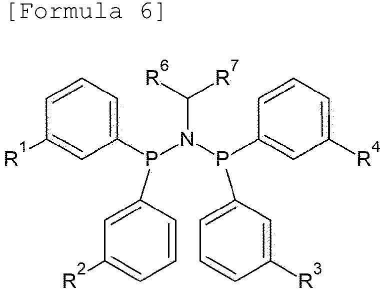

   In Formulas 5 and 6, R¹ to R⁴ are the same as defined in (1) to (8), and R⁶ and R⁷ are each independently an alkyl group of 1 to 27 carbon atoms, where the sum of carbon atoms of R⁶ and R⁷ is 20 to 29.
(10) The present invention provides the ligand compound according to any one among (1) to (9), wherein the ligand compound represented by Formula 1 is represented by any one among Formula 1-1 to Formula 1-12 below. 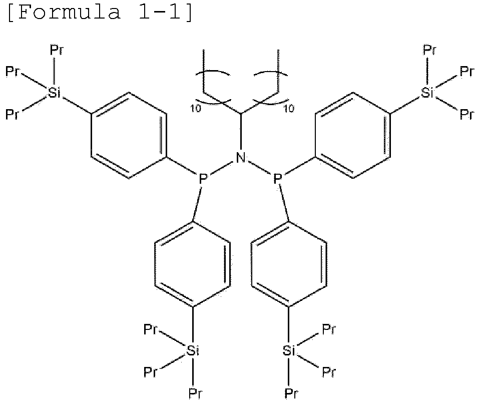 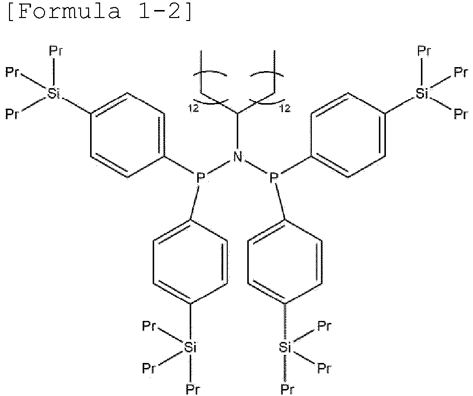 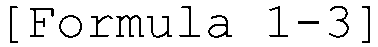 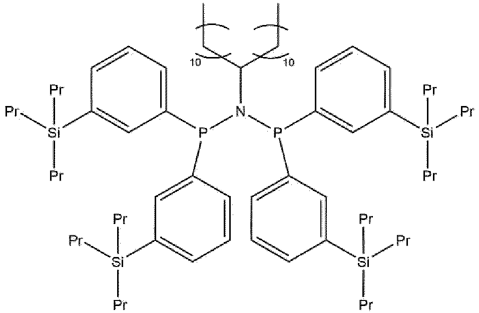 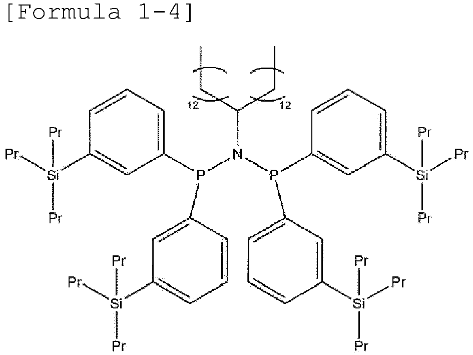 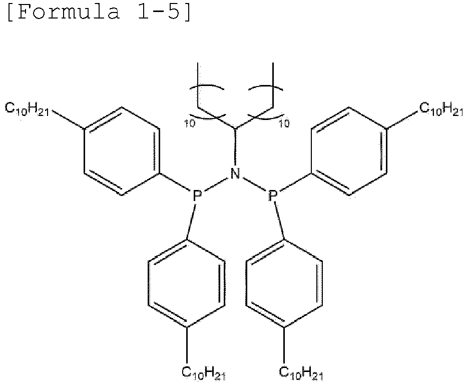 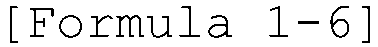 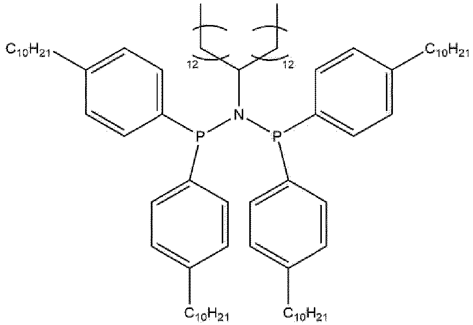 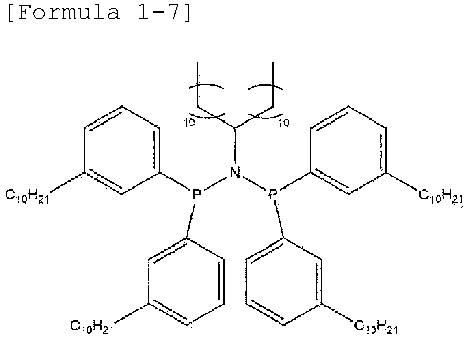 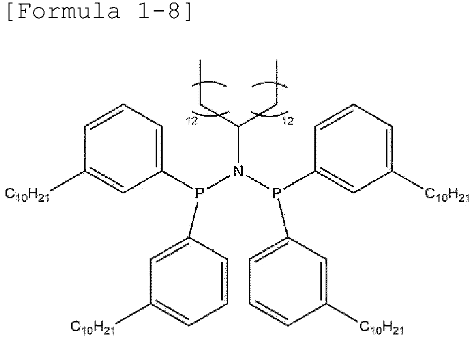 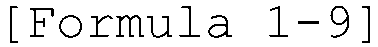 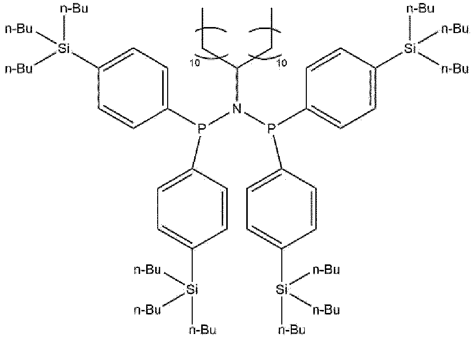 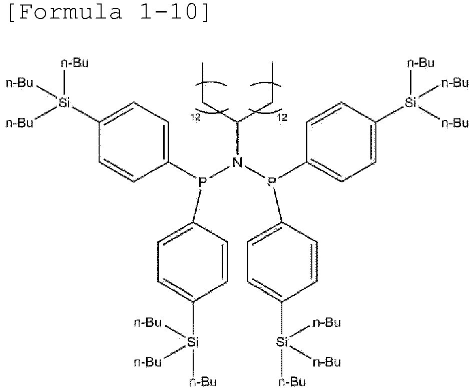 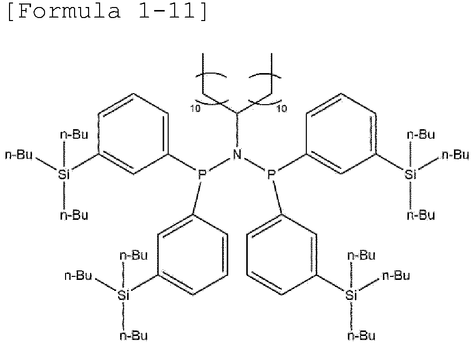 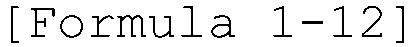 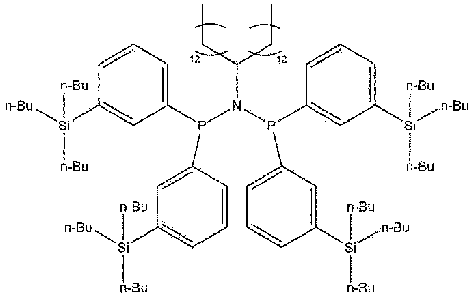
(11) The present invention provides an organochromium compound comprising the ligand compound according to any one among (1) to (10) and chromium making a coordination bond with the ligand compound.
(12) The present invention provides the organochromium compound according to (11), wherein unshared electron pairs of one or more among N and two P in the ligand compound represented by Formula 1 make coordination bonds with the chromium.
(13) The present invention provides a catalyst composition comprising the ligand compound according to any one among (1) to (10), chromium and a cocatalyst.
(14) The present invention provides the catalyst composition according to (13), wherein the chromium is derived from a chromium source, and the chromium source comprises one or more selected from the group consisting of chromium(III) acetylacetonate, chromium(III) chloride tetrahydrofuran, chromium(III) 2-ethylhexanoate, chromium(III) acetate, chromium(III) butyrate, chromium(III) pentanoate, chromium(III) laurate, chromium(III) tris(2,2,6,6-tetramethyl-3,5-heptanedionate) and chromium(III) stearate.
(15) The present invention provides the catalyst composition according to (13) or (14), wherein the cocatalyst is one or more selected from the group consisting of the compounds represented by Formula 10 to Formula 13 below.

   [Formula 10] -[Al(R¹³)-O]ₐ-

   In Formula 10, each R¹³ is independently a halogen group, a hydrocarbyl group of 1 to 20 carbon atoms, or a hydrocarbyl group of 1 to 20 carbon atoms, which is substituted with a halogen group, and a is an integer of 2 or more.

      [Formula 11] E(R¹⁴)₃
   In Formula 11, E is aluminum or boron, and each R¹⁴ is independently hydrogen, a halogen group, a hydrocarbyl group of 1 to 20 carbon atoms, or a hydrocarbyl group of 1 to 20 carbon atoms, which is substituted with a halogen group.

      [Formula 12] [L-H]⁺[G(Y)₄]⁻

      [Formula 13] [L]⁺[G(y)₄]⁻
   In Formulas 12 and 13, L is a neutral or cationic Lewis acid, [L-H]⁺ is a brønsted acid, G is an element in group 13, and each Y is independently a substituted or unsubstituted alkyl group of 1 to 20 carbon atoms or a substituted or unsubstituted aryl group of 6 to 20 carbon atoms, where if the alkyl group or the aryl group is substituted, the substituent is a halogen group, a hydrocarbyl group of 1 to 20 carbon atoms, an alkoxy group of 1 to 20 carbon atoms, or an aryloxy group of 6 to 20 carbon atoms.
(16) The present invention provides a method for preparing a linear alpha-olefin, the method comprising a step (S10) of oligomerizing ethylene in the presence of the catalyst composition according to any one among (13) to (15).
(17) The present invention provides the method for preparing a linear alpha-olefin according to (16), wherein the linear alpha-olefin is 1-hexene, 1-octene or a mixture of them.

### ADVANTAGEOUS EFFECTS

If the oligomerization of ethylene is performed using the organochromium compound including the ligand compound and the catalyst composition of the present invention, a linear alpha-olefin may be produced with excellent productivity and significantly reduced production of the isomers of 1-hexene and 1-octene due to high catalyst activity, thereby producing the linear alpha-olefin in significantly improved selectivity to 1-hexene and 1-octene.

### MODE FOR CARRYING OUT THE INVENTION

Hereinafter, the present invention will be described in more detail to assist the understanding of the present invention.

It will be understood that words or terms used in the present disclosure and claims shall not be interpreted as the meaning defined in commonly used dictionaries. It will be further understood that the words or terms should be interpreted as having a meaning that is consistent with their meaning in the context of the relevant art and the technical idea of the invention, based on the principle that an inventor may properly define the meaning of the words or terms to best explain the invention.

### Ligand Compound

The present invention provides a ligand compound which is applicable to a catalyst used for the oligomerization reaction of ethylene. If the ligand compound is applied to the oligomerization reaction of ethylene, particularly, to a catalyst composition for forming a linear alpha-olefin, excellent catalyst activity may be shown, while showing high selectivity to a linear alpha-olefin. Particularly, if compared to the conventional PNP-based catalyst, the production amount of the isomers of 1-hexene and 1-octene is small under the same reaction conditions, and a linear alpha-olefin may be prepared more efficiently.

According to an embodiment of the present invention, the organochromium compound making a coordination bond with the ligand compound may be utilized for the preparation of a linear alpha-olefin using ethylene, and oligomerization reaction may be performed in the reaction under ethylene conditions to form a liquid type alpha-olefin, particularly, a liquid type 1-hexene or 1-octene with high selectivity. This is due to the increase of the selectivity to an alpha-olefin with a specific length through a transition state forming a metallacycle in the oligomerization reaction of ethylene.

According to an embodiment of the present invention, the ligand compound includes a diphosphino aminyl moiety, and aryl having a specific substituent connected with the terminal of the diphosphino aminyl moiety, and accordingly, the ligand compound itself may have a type which may play the role of a strong electron donating group. Due to such structural features, the ligand compound may be applied to a catalyst system of oligomerizing ethylene to show high activity, particularly, high selectivity to 1-hexene, 1-octene or the like, because the production amount of the isomers of 1-hexene and 1-octene is small. This is considered due to the interaction between adjacent chromium active sites. Particularly, if aryl substituted with a specific substituent is connected with the phosphor (P) atom of diphosphino aminyl, electron density may increase at the phosphor (P) atom and nitrogen (N) atom included in the diphosphino aminyl, and the electrical and three-dimensional properties of the whole ligand compound may change. Accordingly, there may be changes of the bond between the ligand and the chromium atom, the structure of the catalyst may be stabilized even further, an alpha-olefin with higher activity and selectivity may be formed by changing the energy of a transition state (activation energy) when compared to the conventional metallacycloheptane or metallacyclononane type, and the amount of by-products such as a solid alpha-olefin having a large molecular weight like a polyethylene wax (PE wax), may be reduced even further.

According to an embodiment of the present invention, the ligand compound is characterized in that a phenyl positioned at the terminal of the diphosphino aminyl moiety has a silyl group substituted with an alkyl group having a specific carbon number at the meta position or the para position as a substituent. The substituent substituted at the meta position or the para position of the phenyl may increase the solubility of the ligand compound and the metal complex compound in a polymerization solvent, thereby improving the activity and selectivity. Accordingly, if the ligand compound is used, a chromium catalyst with high stability and excellent activity and selectivity may be produced.

According to an embodiment of the present invention, in the ligand compound, to a nitrogen atom bonded to two phosphor atoms, a bulky substituent like an alkyl group of 21 to 30 carbon atoms is bonded, and the bulky substituent bonded to the nitrogen prevents the rotation of the bond between the nitrogen and the phosphor to improve the catalyst stability and activity even further. In this case, according to the steric properties of the substituent bonded to the nitrogen atom, the activity, stability, selectivity or the like of the catalyst may change. If the steric strain of the substituent bonded to the nitrogen atom is too high, there are problems in that the synthesis of a ligand and the formation of a metal complex compound is difficult, and the complex compound produced becomes unstable. In addition, if the steric strain of the substituent bonded to the nitrogen atom is too high, there are problems in that the approach of a raw material such as ethylene becomes difficult, and the activity of the catalyst is deteriorated. In addition, if the steric strain of the substituent bonded to the nitrogen atom is too low, the rotation of the bond between the nitrogen atom and the phosphor atom may not be prevented, and a metal central atom may not be protected, to be a factor of deteriorating the activity and stability of the catalyst. That is, if the steric strain of the substituent bonded to the nitrogen atom is too high or too low, there are problems in that the activity of the catalyst is reduced, the stability is deteriorated, and the production amount of by-products like polyethylene wax increases.

In particular, in the steric strain of the substituent bonded to the nitrogen atom, if the substituent is an alkyl group of 20 or less carbon atoms, the steric strain is not sufficiently large, so it is difficult to exhibit a sufficient effect in reducing the isomers of 1-hexene and 1-octene. In addition, if the substituent is an alkyl group of 31 or more carbon atoms, the ratio of the isomers of 1-hexene and 1-octene may decrease, but the steric strain becomes too large, making it difficult for a substrate to approach, thereby decreasing the activity of the catalyst. Therefore, if the substituent bonded to the nitrogen atom is an alkyl group of 21 or more and 30 or less carbon atoms, the most appropriate steric strain may be formed, and thus the production ratio of the isomers of 1-hexene and 1-octene may be reduced without decreasing the activity of the catalyst. Furthermore, the ratio of the isomers of 1-hexene and 1-octene thus reduced may reduce the number of columns in the process of purifying the product, and may also reduce the energy cost used in the purification.

According to an embodiment of the present invention, the ligand compound may be represented by Formula 1 below.

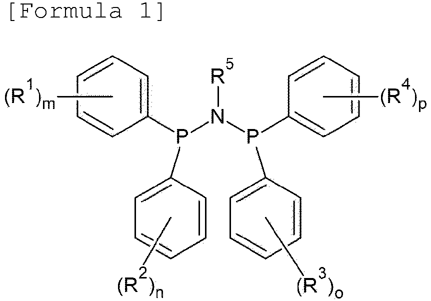

In Formula 1, R¹ to R⁴ are each independently an alkyl group of 1 to 20 carbon atoms, a cycloalkyl group of 5 to 20 carbon atoms, an alkoxy group of 1 to 20 carbon atoms, or - Si(R⁷)₃, m, n, o, and p are each independently 0 or an integer selected from 1 to 5, where if m, n, o, and p are 2 or more, a plurality of R¹ to R⁴ are the same or different from each other, R⁵ is an alkyl group of 21 to 30 carbon atoms, and R⁷ is an alkyl group of 1 to 20 carbon atoms, where a plurality of R⁷ are the same or different from each other.

In the present invention, an "alkyl group" means a hydrocarbon moiety of a linear or branched type, and particularly includes a methyl group, an ethyl group, a n-propyl group, an isopropyl group, a n-butyl group, an isobutyl group, a t-butyl group, a n-pentyl group, an isopentyl group and a hexyl group, according to the carbon number defined.

In the present invention, a "cycloalkyl group" may mean a cyclic hydrocarbon moiety, and as specific examples, it may be a cyclopentyl group, a cyclohexyl group, a cycloheptyl group, a cyclooctyl group or the like, depending on the carbon number defined.

In the present invention, a "trialkylsilyl group" means a substituent represented by -SiR₃ where each R is independently an alkyl group, and the carbon number of the trialkylsilyl group, if referred to, may mean the sum of all the carbon numbers of R.

In the present invention, "aryl" refers to an optionally substituted benzene ring, or refers to a ring system which may be formed by fusing one or more optional substituents, unless otherwise referred to. Examples of the optional substituent include a substituted alkyl group of 1 to 2 carbon atoms, a substituted alkenyl group of 2 to 3 carbon atoms, a substituted alkynyl group of 2 to 3 carbon atoms, a heteroaryl group, a heterocyclic group, an aryl group, an alkoxy group having optionally 1 to 3 fluorine substituents, aryloxy, aralkoxy, acyl, aroyl, heteroaroyl, acyloxy, aroyloxy, heteroaroyloxy, sulfanyl, sulfinyl, sulfonyl, aminosulfonyl, sulfonylamino, carboxyamide, aminocarbonyl, carboxy, oxo, hydroxyl, mercapto, amino, nitro, cyano, halogen or ureido. Such a ring or ring system may be optionally fused with an aryl ring (for example, benzene ring), carbocyclic ring or heterocyclic ring, having optionally one or more substituents. Non-limiting examples include phenyl, naphthyl, tetrahydronaphthyl, biphenyl, indanyl, anthracyl, phenanthryl and substituted derivatives thereof.

According to an embodiment of the present invention, R¹ to R⁴ may be each independently an alkyl group of 1 to 20 carbon atoms, a cycloalkyl group of 5 to 20 carbon atoms, an alkoxy group of 1 to 20 carbon atoms, or -Si(R⁷)₃. As a specific example, R¹ to R⁴ may be each independently an alkyl group of 1 to 10 carbon atoms, a cycloalkyl group of 5 to 10 carbon atoms, an alkoxy group of 1 to 10 carbon atoms, or - Si(R⁷)₃, and as a more specific example, R¹ to R⁴ may be each independently -Si(R⁷)₃. In this case, R⁷ is an alkyl group of 1 to 20 carbon atoms, and a plurality of R⁷ may be the same or different from each other. As a specific example, R⁷ may be an alkyl group of 1 to 10 carbon atoms, and as a more specific example, R⁷ may be an alkyl group of 1 to 5 carbon atoms.

According to an embodiment of the present invention, R¹ to R⁴ may be each independently a trimethylsilyl group, a triethylsilyl group, a tri-n-propylsilyl group, a tri-n-butylsilyl group, a tri-n-octylsilyl group, or a dimethyl(n-octyl)silyl group. That is, the ligand compound has a silyl group substituted with an alkyl group having a specific carbon number as a substituent, at the meta position or the para position of a phenyl group positioned at the terminal of the diphosphino aminyl moiety.

According to an embodiment of the present invention, m, n, o, and p may be each independently 0 or an integer selected from 1 to 5, and if m, n, o, and p are 2 or more, a plurality of R¹ to R⁴ may be the same as or different from each other. As a specific example, m, n, o, and p may be each independently 0, 1, or 2, and as a more specific example, m, n, o, and p may be 1.

According to an embodiment of the present invention, R⁵ may be an alkyl group of 21 to 30 carbon atoms, and as a specific example, R⁵ may be a branched alkyl group of 21 to 30 carbon atoms. As a more specific example, R⁵ may be a branched alkyl group of 21 or more, 22 or more, or 23 or more carbon atoms, and further may be a branched alkyl group of 30 or less, 29 or less, 28 or less, or 27 or less carbon atoms.

According to an embodiment of the present invention, the ligand compound represented by Formula 1 may be represented by Formula 2 or Formula 3 below.

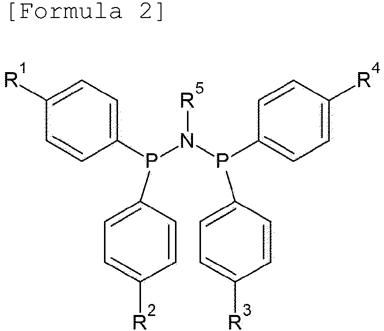

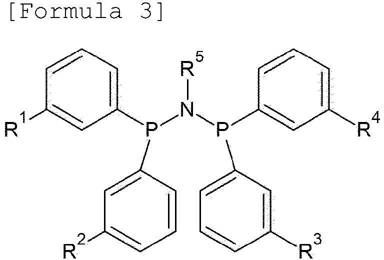

In Formula 2 and Formula 3, R¹ to R⁴ are the same as defined above.

According to an embodiment of the present invention, the ligand compound represented by Formula 1 may be represented by Formula 4 below.

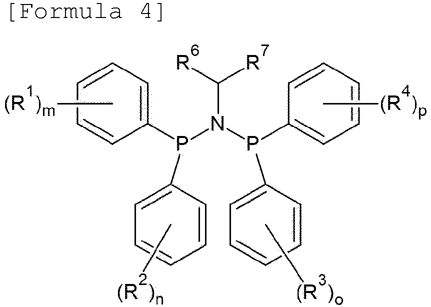

In Formula 4, R¹ to R⁴, m, n, o and p are the same as defined above. In addition, R⁶ and R⁷ are each independently an alkyl group of 1 to 27 carbon atoms, where the sum of carbon atoms of R⁶ and R⁷ may be 20 to 29. As a specific example, R⁶ and R⁷ may be each independently an alkyl group of 7 to 17 carbon atoms, and the sum of the carbon atoms of R⁶ and R⁷ may be 20 to 29. As a more specific example, R⁶ and R⁷ may be each independently an alkyl group of 7 or more, 8 or more, 9 or more, 10 or more, or 11 or more carbon atoms, and further may be an alkyl group of 17 or less, 16 or less, 15 or less, 14 or less, or 13 or less carbon atoms, and in this case, the sum of the carbon atoms of R⁶ and R⁷ may be 20 to 29.

According to an embodiment of the present invention, the ligand compound represented by Formula 1 may be represented by Formula 5 or Formula 6 below.

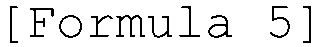

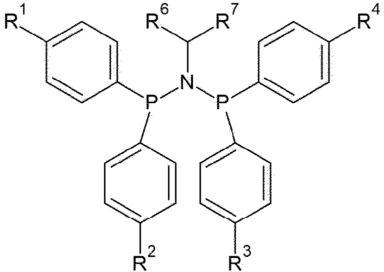

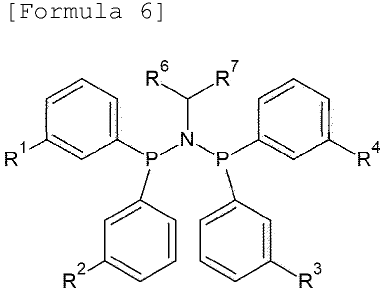

In Formulas 5 and 6, R¹ to R⁴, R⁶ and R⁷ are the same as defined above.

According to an embodiment of the present invention, the ligand compound represented by Formula 1 may be represented by any one among Formula 1-1 to Formula 1-12 below.

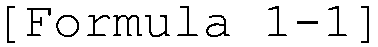

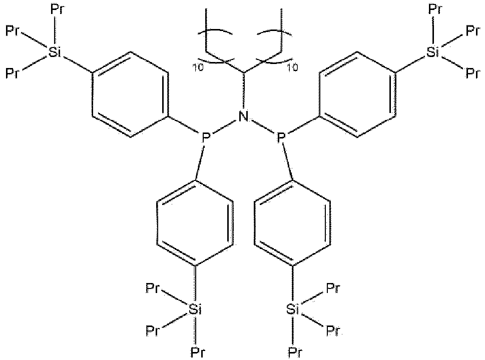

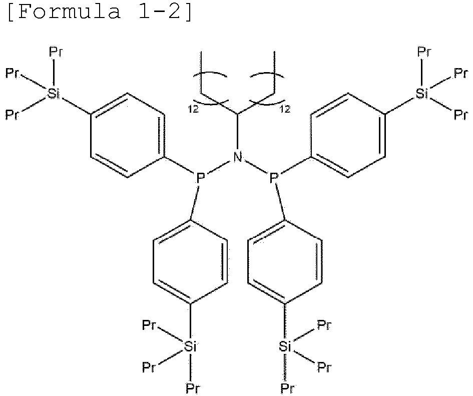

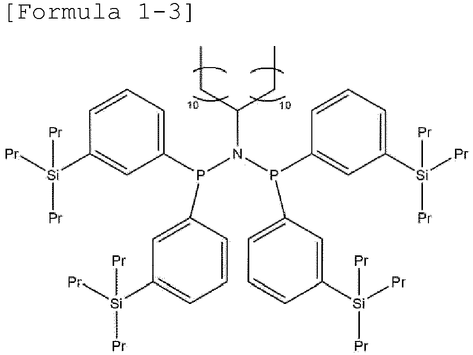

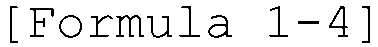

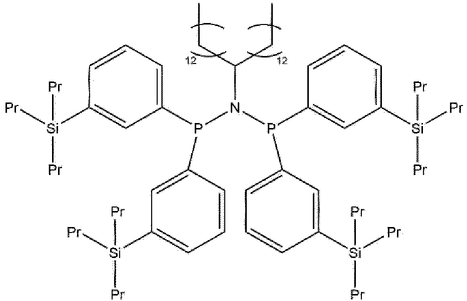

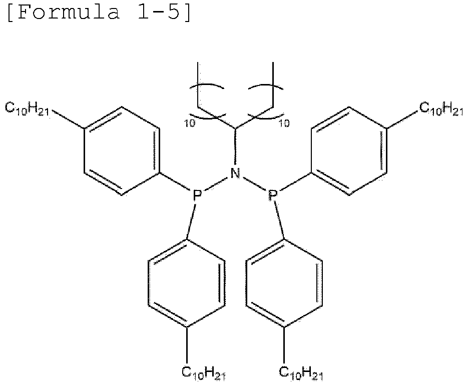

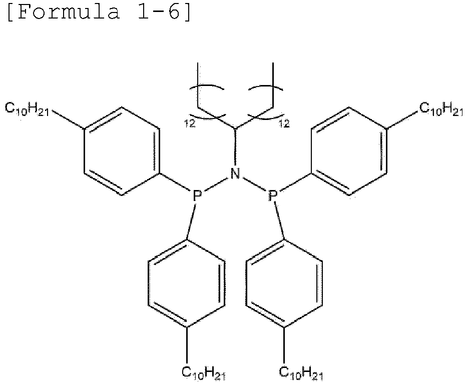

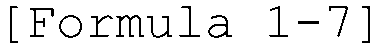

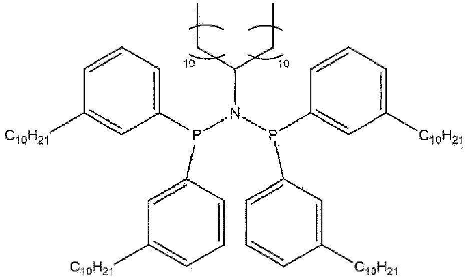

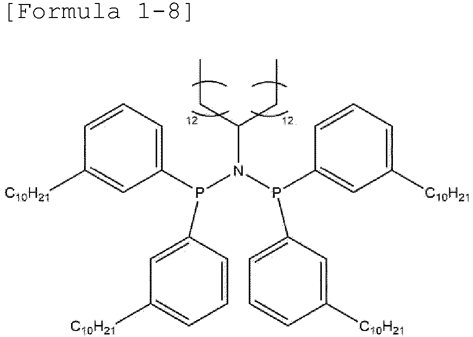

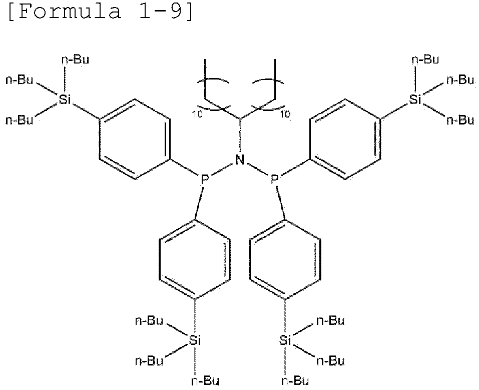

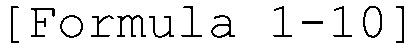

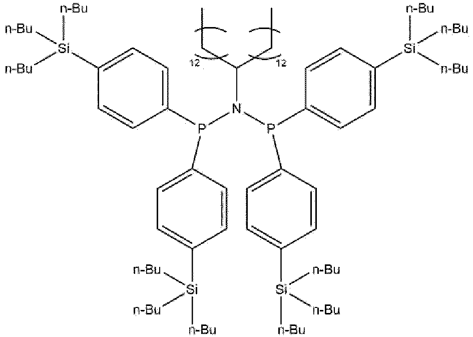

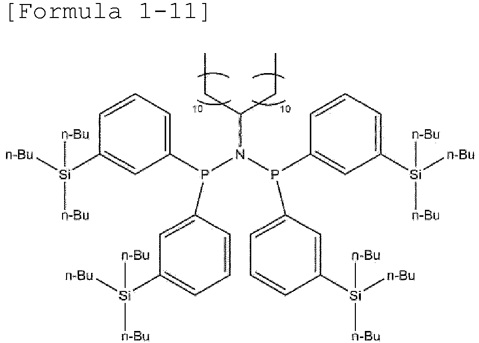

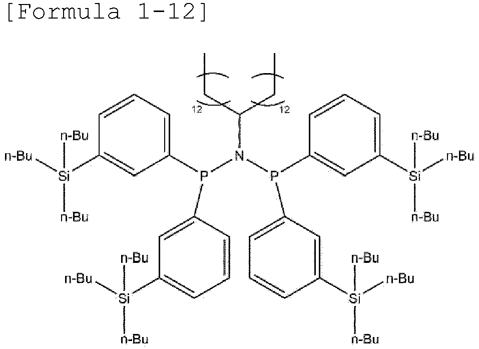

According to an embodiment of the present invention, the ligand compound may be accomplished by various combinations within a range satisfying the above-described conditions in addition to the above embodiments, and any compounds may be applicable as the ligand compound of the present invention as long as it is represented by Formula 1.

### Organochromium Compound and Catalyst Composition

The present invention provides an organochromium compound including the ligand compound represented by Formula 1 and chromium (Cr) which makes a coordination bond with the ligand compound.

According to an embodiment of the present invention, the organochromium compound is the chromium complex compound of the ligand compound and may have a type where the chromium of a chromium source makes coordination bonds with unshared electron pairs of one or more among N and two P in the ligand compound represented by Formula 1. That is, in the structure, the phosphor atom or nitrogen atom of a diphosphino aminyl moiety provides the chromium atom with an unshared electron pair, and particularly, a bidentated state in which two unshared electron pairs are shared, may be preferable. Such an organochromium compound may be applied to a catalyst system for the oligomerization reaction of ethylene to show excellent catalyst activity and high selectivity to 1-hexene and 1-octene.

In the present invention, a "catalyst composition" means a state obtainable as a catalyst composition having activity by adding three components including a chromium source, a ligand compound and a cocatalyst, or two components of a transition metal compound and a cocatalyst simultaneously or in an arbitrary order. Here, the catalyst composition may be referred to as a catalyst system, and in the present invention, the catalyst composition and the catalyst system show the same meaning. The three components or two components of the catalyst composition may be added in the presence or absence of a solvent and a monomer, and may be used in a supported or unsupported state.

The present invention provides a catalyst compound including the ligand compound, chromium and a cocatalyst. The ligand compound represented by Formula 1 and chromium may be coordinated as described above to form an organochromium compound. That is, the catalyst system may be a three-component catalyst system including chromium, the ligand compound represented by Formula 1 and a cocatalyst, or a two-component catalyst system including the organochromium compound and a cocatalyst. In a particular embodiment, the catalyst composition may include an organochromium compound including the ligand compound and chromium making a coordination bond with the ligand compound, and a cocatalyst. In addition, the catalyst composition may include a chromium compound in which a partial component of the cocatalyst is bonded to the organochromium compound.

According to an embodiment of the present invention, the chromium may be derived from a chromium source, and the chromium source may be an organic or inorganic chromium compound with an oxidation state of 0 to 6. In a particular embodiment, the chromium source may be a chromium metal, or a compound in which an arbitrary organic or inorganic radical is bonded to chromium. Here, the organic radical may be alkyl, alkoxy, ester, ketone, amido, carboxylate radical or the like, having 1 to 20 carbon atoms per radical, and the inorganic radical may be halide, sulfate, oxide or the like.

According to an embodiment of the present invention, the chromium source is a compound showing high activity for the oligomerization of an olefin and easily used and obtained, and may be one or more compounds selected from the group consisting of chromium(III) acetylacetonate, chromium(III) chloride tetrahydrofuran, chromium(III) 2-ethylhexanoate, chromium(III) acetate, chromium(III) butyrate, chromium(III) pentanoate, chromium(III) laurate, chromium(III) tris(2,2,6,6-tetramethyl-3,5-heptanedionate) and chromium(III) stearate.

According to an embodiment of the present invention, the cocatalyst may be one or more selected from the group consisting of the compounds represented by Formula 10 to Formula 13 below.

[Formula 10] -[Al(R¹³)-O]ₐ-

In Formula 10, each R¹³ is independently a halogen group, a hydrocarbyl group of 1 to 20 carbon atoms, or a hydrocarbyl group of 1 to 20 carbon atoms, which is substituted with a halogen group, and a is an integer of 2 or more.

[Formula 11] E(R¹⁴)₃

In Formula 11, E is aluminum or boron, and each R¹⁴ is independently hydrogen, a halogen group, a hydrocarbyl group of 1 to 20 carbon atoms, or a hydrocarbyl group of 1 to 20 carbon atoms, which is substituted with a halogen group.

[Formula 12] [L-H]⁺[G(Y)₄]⁻

[Formula 13] [L]⁺[G(Y)₄]⁻

In Formula 12 and Formula 13, L is a neutral or cationic Lewis acid, [L-H]⁺ is a brønsted acid, G is an element in group 13, and each Y is independently a substituted or unsubstituted alkyl group of 1 to 20 carbon atoms, or a substituted or unsubstituted aryl group of 6 to 20 carbon atoms, where if the alkyl group or the aryl group is substituted, the substituent is a halogen group, a hydrocarbyl group of 1 to 20 carbon atoms, an alkoxy group of 1 to 20 carbon atoms, or an aryloxy group of 6 to 20 carbon atoms.

According to an embodiment of the present invention, the catalyst composition may be prepared by numbers of methods. In a particular embodiment, first, the catalyst composition may be prepared by including a step of contacting the organochromium compound with the compound represented by Formula 10 or Formula 11. Second, the catalyst composition may be prepared by including a step of contacting the organochromium compound with the compound represented by Formula 10 or Formula 11 to obtain a mixture; and a step of adding the compound represented by Formula 12 or Formula 13 to the mixture. Third, the catalyst composition may be prepared by including a step of contacting the organochromium compound with the compound represented by Formula 12 or Formula 13. Fourth, the catalyst composition may be prepared by including a step of contacting the organochromium compound with the compound represented by Formula 12 or Formula 13 to obtain a mixture; and a step of adding the compound represented by Formula 10 or Formula 11 to the mixture. Fifth, the catalyst composition may be prepared by including a step of contacting the chromium source with the compound represented by Formula 12 or Formula 13 to obtain a reaction product, and a step of contacting the reaction product with the ligand compound.

According to an embodiment of the present invention, in the first method or the third method among the preparation methods of the catalyst composition, the molar ratio of the compound represented by Formula 10 or Formula 11 in contrast to the organochromium compound may be 1: 2 to 5,000, particularly, 1: 100 to 3,000, more particularly, 1: 300 to 1,500, and within this range, the alkylation of the organochromium compound may be completely performed, the activity of the catalyst composition may be improved, the degradation of the activation of the alkylated organochromium compound due to the side reactions between remaining alkylating agents, may be prevented, and at the same time, the economic feasibility and the purity of a linear alpha-olefin prepared may be improved.

According to an embodiment of the present invention, in the case of the second method among the preparation methods of the catalyst composition, the molar ratio of the compound represented by Formula 12 or Formula 13 in contrast to the organochromium compound may be 1: 1 to 500, particularly, 1: 1 to 50, more particularly, 1: 1 to 1: 25, and within this range, the amount of an activating agent is sufficient, the activation of a metal compound may be completely performed, and the activity of the catalyst composition may be improved, the activating agent remained may be minimized, and the economic feasibility and the purity of a linear alpha-olefin prepared may be improved.

According to an embodiment of the present invention, the compound represented by Formula 10 may be alkylaluminoxane, and particular examples may include methylaluminoxane, ethylaluminoxane, isobutylaluminoxane, butylaluminoxane or the like, more particularly, methylaluminoxane.

According to an embodiment of the present invention, the compound represented by Formula 11 may be trialkyl aluminum, dialkyl aluminum halide, alkyl aluminum dihalide, dialkyl aluminum hydride, alkyl aluminum dihydride, trialkylboron or the like. In a particular embodiment, the compound represented by Formula 11 may be trialkyl aluminum such as trimethylaluminum, triethylaluminum, triisobutylaluminum, tripropylaluminum, tributylaluminum, triisopropylaluminum, tri-s-butylaluminum, tricyclopentylaluminum, tripentylaluminum, triisopentylaluminum, trihexylaluminum, trioctylaluminum, ethyldimethylaluminum, methyldiethylaluminum, triphenylaluminum, and tri-p-tolylaluminum; dialkylaluminum halide such as diethylaluminum chloride; dialkyl aluminum hydride such as diethyl aluminum hydride, di-n-propyl aluminum hydride, diisopropyl aluminum hydride, di-n-butyl aluminum hydride, dibutyl aluminum hydride, diisobutyl aluminum hydride (DIBAH), di-n-octyl aluminum hydride, diphenyl aluminum hydride, di-p-tolyl aluminum hydride, dibenzyl aluminum hydride, phenylethyl aluminum hydride, phenyl-n-propyl aluminum hydride, phenylisopropyl aluminum hydride, phenyl-n-butyl aluminum hydride, phenylisobutyl aluminum hydride, phenyl-n-octyl aluminum hydride, p-tolylethyl aluminum hydride, p-tolyl-n-propyl aluminum hydride, p-tolylisopropyl aluminum hydride, p-tolyl-n-butyl aluminum hydride, p-tolylisobutyl aluminum hydride, p-tolyl-n-octyl aluminum hydride, benzylethyl aluminum hydride, benzyl-n-propyl aluminum hydride, benzylisopropyl aluminum hydride, benzyl-n-butyl aluminum hydride, benzylisobutyl aluminum hydride and benzyl-n-octyl aluminum hydride; alkyl aluminum dihydride such as n-propyl aluminum dihydride, isopropyl aluminum dihydride, n-butyl aluminum dihydride, isobutyl aluminum dihydride, and n-octylaluminum dihydride; and trialkylboron such as trimethylboron, triethylboron, triisobutylboron, tripropylboron and tributylboron.

According to an embodiment of the present invention, the compound represented by Formula 12 or Formula 13 may be trimethylammonium tetraphenylborate, triethylammonium tetraphenylborate, tripropylammonium tetraphenylborate, tributylammonium tetraphenylborate, methyl dioctadecylammonium tetraphenylborate, N,N-dimethylanilinium tetraphenylborate, N,N-diethylanilinium tetraphenylborate, N,N-dioctadecylanilinium tetraphenylborate, trimethylammonium tetra(p-tolyl)borate, triethylammonium tetra(p-tolyl)borate, tripropylammonium tetra(p-tolyl)borate, tributylammonium tetra(p-tolyl)borate, methyl dioctadecylammonium tetra(p-tolyl)borate, N,N-dimethylanilinium tetra(p-tolyl)borate, N,N-diethylanilinium tetra(p-tolyl)borate, N,N-dioctadecylanilinium tetra(p-tolyl)borate, trimethylammonium tetra(o,p-dimethylphenyl)borate, triethylammonium tetra(o,p-dimethylphenyl)borate, tripropylammonium tetra(o,p-dimethylphenyl)borate, tributylammonium tetra(o,p-dimethylphenyl)borate, methyl dioctadecylammonium tetra(o,p-dimethylphenyl)borate, N,N-dimethylanilinium tetra(o,p-dimethylphenyl)borate, N,N-diethylailinium tetra(o,p-dimethylphenyl)borate, N,N-dioctadecylanilinium tetra(o,p-dimethylphenyl)borate, trimethylammonium tetrakis(p-trifluoromethylphenyl)borate, triethylammonium tetrakis(p-trifluoromethylphenyl)borate, tripropylammonium tetrakis(p-trifluoromethylphenyl)borate, tributylammonium tetrakis(p-trifluoromethylphenyl)borate, methyl dioctadecylammonium tetrakis(p-trifluoromethylphenyl)borate, N,N-dimethylanilinium tetrakis(p-trifluoromethylphenyl)borate, N,N-diethylailinium tetrakis(p-trifluoromethylphenyl)borate, N,N-dioctadecylanilinium tetrakis(p-trifluoromethylphenyl)borate, trimethylammonium tetrakis(pentafluorophenyl)borate, triethylammonium tetrakis(pentafluorophenyl)borate, tripropylammonium tetrakis(pentafluorophenyl)borate, tributylammonium tetrakis(pentafluorophenyl)borate, methyl dioctadecylammonium tetrakis(pentafluorophenyl)borate, N,N-dimethylanilinium tetrakis(pentafluorophenyl)borate, N,N-diethylanilinium tetrais(pentafluorophenyl)borate, N,N-dioctadecylanilinium tetrakis(pentafluorophenyl)borate, trimethylphosphonium tetraphenylborate, triethylphosphonium tetraphenylborate, tripropylphosphonium tetraphenylborate, tributylphosphonium tetraphenylborate, trimethylcarbonium tetraphenylborate, triethylcarbonium tetraphenylborate, tripropylcarbonium tetraphenylborate, tributylcarbonium tetraphenylborate, trimethylammonium tetraphenylaluminate, triethylammonium tetraphenylaluminate, tripropylammonium tetraphenylaluminate, tributylammonium tetraphenylaluminate, trimethylammonium tetra(p-tolyl)aluminate, triethylammonium tetra(p-tolyl)aluminate, tripropylammonium tetra(p-tolyl)aluminate, tributylammonium tetra(p-tolyl)aluminate or the like.

According to an embodiment of the present invention, the amount ratio of the components constituting the catalyst composition may be determined considering the catalyst activity and the selectivity to a linear alpha-olefin. In a particular embodiment, if the catalyst composition is a three-component catalyst composition, the molar ratio of diphosphino aminyl moiety: chromium source: cocatalyst of the ligand compound may be controlled to about 1: 1: 1 to about 10: 1: 10,000, or about 1: 1: 100 to 5: 1: 3,000. In addition, if the catalyst composition is a two-component catalyst composition, the molar ratio of diphosphino aminyl moiety: cocatalyst of the organochromium compound may be controlled to 1: 1 to 1: 10,000, or 1: 1 to 1: 5,000 or 1: 1 to 1: 3,000.

According to an embodiment of the present invention, the reaction solvent during preparing the catalyst composition may use a hydrocarbon-based solvent such as pentane, hexane and heptane; and an aromatic solvent such as benzene and toluene.

According to an embodiment of the present invention, the components constituting the catalyst composition may be added simultaneously or in an arbitrary order in the presence or absence of a suitable solvent and monomer, and may act as a catalyst composition having activity. In this case, the suitable solvent may include heptane, toluene, cyclohexane, methylcyclohexane, 1-hexene, 1-octene, diethyl ether, tetrahydrofuran, acetonitrile, dichloromethane, chloroform, chlorobenzene, methanol, acetone or the like.

According to an embodiment of the present invention, the organochromium compound and the cocatalyst may be used in supported types in supports, and in this case, the support may be silica or alumina.

According to an embodiment of the present invention, the catalyst composition may further include a support. In a particular embodiment, the ligand compound represented by Formula 1 may be applied for the oligomerization of ethylene, in a supported type in a support. The support may be a metal, a metal salt or a metal oxide, applied in a supported catalyst, and in a particular embodiment, the support may be silica, silica-alumina, silica-magnesia or the like, and may include the oxide, carbonate, sulfate, or nitrate component of a metal such as Na₂O, K₂CO₃, BaSO₄, and Mg(NO₃)₂.

According to an embodiment of the present invention, the catalyst composition may be used for the trimerization or tetramerization reaction of ethylene, and may produce 1-hexene or 1-octene with high selectivity by the above-description.

### Method for Oligomerizing Ethylene

The present invention provides a method for preparing a linear alpha-olefin as a method for oligomerizing ethylene, comprising a step of oligomerizing ethylene in the presence of the catalyst composition (S10).

In the present invention, "oligomerization" means oligomerizing olefins. According to the number of olefins polymerized, the oligomerization is referred to as trimerization or tetramerization, and is collectively referred to as multimerization. Particularly, in the present disclosure, the oligomerization may mean selective preparation of 1-hexene and 1-octene, which are main comonomers of a LLDPE from ethylene.

According to an embodiment of the present invention, the oligomerization reaction of ethylene may be trimerization or tetramerization of ethylene, and 1-hexene or 1-octene may be formed as the resultant reaction product, and accordingly, the linear alpha-olefin may be 1-hexene, 1-octene or a mixture thereof.

According to an embodiment of the present invention, the method of oligomerizing ethylene may be performed by using ethylene as a raw material and applying the above-described catalyst composition, a common apparatus and contacting technique. In a particular embodiment, the oligomerization reaction of ethylene may include homogeneous liquid phase reaction in the presence or absence of an inert solvent, slurry reaction by which a portion or whole of the catalyst composition is not dissolved, bulk phase reaction in which an alpha-olefin product acts as a main medium, or gas phase reaction.

According to an embodiment of the present invention, the oligomerization reaction of ethylene may be performed under an inert solvent. Particular examples of the inert solvent may include benzene, toluene, xylene, cumene, chlorobenzene, dichlorobenzene, heptane, cyclohexane, methylcyclohexane, methylcyclopentane, n-hexane, 1-hexene, 1-octene, 2,2,4-trimethylpentane or the like.

According to an embodiment of the present invention, the oligomerization reaction of ethylene may be performed under a temperature of 0°C to 200°C, or 0°C to 150°C, or 30°C to 100°C, or 50°C to 100°C. In addition, the reaction may be performed under a pressure of 15 psig to 3000 psig, or 15 psig to 1500 psig, or 15 psig to 1000 psig.

Hereinafter, embodiments of the present invention will be explained in more detail so that a person skilled in the art to which the present invention belongs, may easily perform. However, the embodiments may be accomplished by various different types and is not limited to the embodiments explained herein.

### Synthetic Examples and Comparative Synthetic Examples

### Synthetic Example 1: Synthesis of Ligand Compound Represented by Formula 1-1

20 mmol (2 eq) of 1-bromo-4-(tripropylsilyl)benzene was dissolved in 20 ml of tetrahydrofuran, and the solution was cooled to -78°C. 20 mmol (2 eq) of n-butyllithium was added thereto dropwise while maintaining the temperature, followed by stirring for 3 hours. Then, 10 mmol (1 eq) of dichloro(diethylamino)phosphine dissolved in 10 ml of tetrahydrofuran was added thereto dropwise, and the temperature was raised to room temperature, followed by stirring overnight. Thereafter, the solvent was removed using vacuum. The first intermediate thus obtained without further purification was dissolved in 30 ml of n-hexane, and then HCl (in ether, 2 eq) was added thereto, followed by stirring for 15 minutes and filtering. The filtrate was dried in vacuum. 2.1 mmol (2.1 eq) of the second intermediate thus obtained was dissolved in 3.8 ml of dichloromethane, and then 3 mmol (3 eq) of triethylamine was added thereto. Then, 1 mmol (1 eq) of tricosan-12-amine dissolved in dichloromethane was slowly added thereto, and stirred at room temperature overnight. After removing the solvent in vacuum, the resultant was dissolved in 7.6 ml of n-hexane, and loaded onto the top of a silica column chromatography. By using n-hexane containing 1 wt% of triethylamine, silica filtration was performed, and the solution thus obtained was concentrated to obtain a ligand compound represented by Formula 1-1.

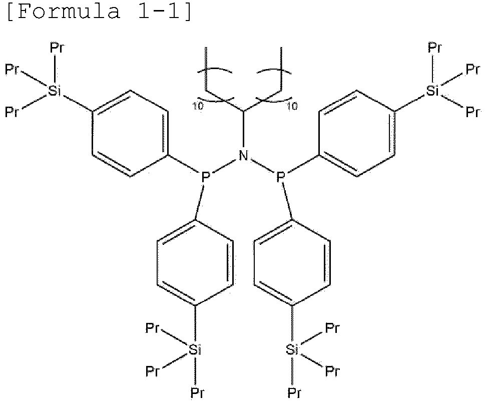

### N-(bis(4-(tripropylsilyl)phenyl)phosphaneyl)-N-(tricosan-12-yl)-1,1-bis(4-(tripropylsilyl)phenyl)phosphanamine

¹H NMR (500 MHz, CDCl₃): δ 7.44(m, 8H), 7.09(m, 8H), 2.50(m, 1H), 1.50(m, 4H), 1.48(m, 24H), 1.38(m, 4H), 1.35(m, 24H), 1.34(m, 32H), 1.03(m, 36H), 0.92(m, 6H)

### Synthetic Example 2: Synthesis of Ligand Compound Represented by Formula 1-2

The same method as Synthetic Example 1 was performed except that 1 mmol (1 eq) of heptacosan-14-amine was used instead of 1 mmol (1 eq) of tricosan-12-amine in Synthetic Example 1 to obtain a ligand compound represented by Formula 1-2.

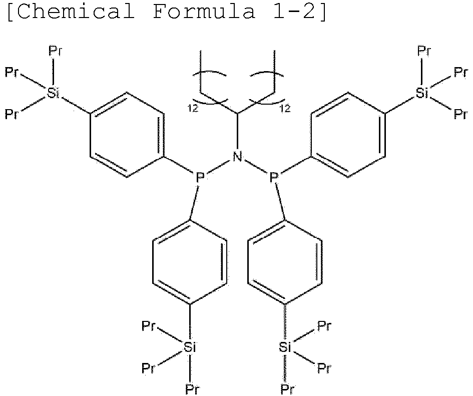

### N-(bis(4-(tripropylsilyl)phenyl)phosphaneyl)-N-(heptacosan-14-yl)-1,1-bis(4-(tripropylsilyl)phenyl)phosphanamine

¹H NMR (500 MHz, CDCl₃): δ 7.41(m, 8H), 7.10(m, 8H), 2.50(m, 1H), 1.49(m, 24H), 1.48(m, 4H), 1.38(m, 24H), 1.36(m, 4H), 1.30(m, 40H), 1.03(m, 36H), 0.91(m, 6H)

### Synthetic Example 3: Synthesis of Ligand Compound Represented by Formula 1-3

The same method as Synthetic Example 1 was performed except that 20 mmol (2 eq) of 1-bromo-3-(tripropylsilyl)benzene was used instead of 20 mmol (2 eq) of 1-bromo-4-(tripropylsilyl)benzene in Synthetic Example 1 to obtain a ligand compound represented by Formula 1-3.

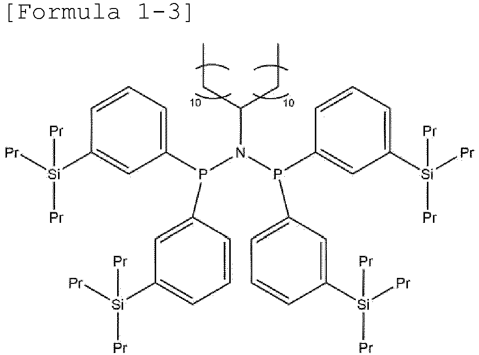

### N-(bis(3-(tripropylsilyl)phenyl)phosphaneyl)-N-(tricosan-12-yl)-1,1-bis(3-(tripropylsilyl)phenyl)phosphanamine

¹H NMR (500 MHz, CDCl₃): δ 7.57(m, 4H), 7.44(m, 4H), 7.33(m, 4H), 7.17(m, 4H), 2.53(m, 1H), 1.52(m, 4H), 1.45(m, 24H), 1.35(m, 28H), 1.30(m, 32H), 1.06(m, 36H), 0.95(m, 6H)

### Synthetic Example 4: Synthesis of Ligand Compound Represented by Formula 1-4

The same method as Synthetic Example 1 was performed except that 20 mmol (2 eq) of 1-bromo-3-(tripropylsilyl)benzene was used instead of 20 mmol (2 eq) of 1-bromo-4-(tripropylsilyl)benzene, and 1 mmol (1 eq) of heptacosan-14-amine was used instead of 1 mmol (1 eq) of tricosan-12-amine in Synthetic Example 1 to obtain a ligand compound represented by Formula 1-4.

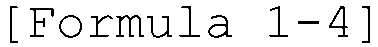

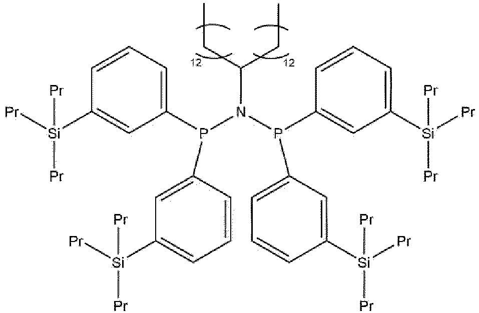

### N-(bis(3-(tripropylsilyl)phenyl)phosphaneyl)-N-(heptacosan-14-yl)-1,1-bis(3-(tripropylsilyl)phenyl)phosphanamine

¹H NMR (500 MHz, CDCl₃): δ 7.65(m, 4H), 7.43(m, 4H), 7.36(m, 4H), 7.10(m, 4H), 2.52(m, 1H), 1.53(m, 4H), 1.51(m, 24H), 1.40(m, 4H), 1.32(m, 24H), 1.29(m, 40H), 1.03(m, 36H), 0.93(m, 6H)

### Synthetic Example 5: Synthesis of Ligand Compound Represented by Formula 1-5

The same method as Synthetic Example 1 was performed except that 20 mmol (2 eq) of 1-bromo-4-decylbenzene was used instead of 20 mmol (2 eq) of 1-bromo-4-(tripropylsilyl)benzene in Synthetic Example 1 to obtain a ligand compound represented by Formula 1-5.

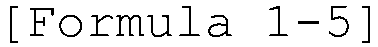

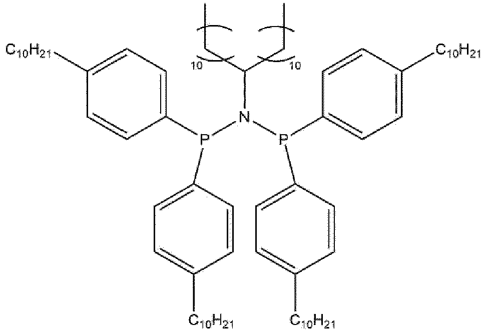

### N-(bis(4-decylphenyl)phosphaneyl)-1,1-bis(4-decylphenyl)-N-(tricosan-12-yl)phosphanamine

¹H NMR (500 MHz, CDCl₃): δ 7.26(m, 8H), 7.20(m, 8H), 2.67(m, 8H), 2.44(m, 1H), 1.64(m, 8H), 1.51(m, 4H), 1.38(m, 4H), 1.30(m, 88H), 0.91(m, 18H)

### Synthetic Example 6: Synthesis of Ligand Compound Represented by Formula 1-6

The same method as Synthetic Example 1 was performed except that 20 mmol (2 eq) of 1-bromo-4-decylbenzene was used instead of 20 mmol (2 eq) of 1-bromo-4-(tripropylsilyl)benzene, and 1 mmol (1 eq) of heptacosan-14-amine was used instead of 1 mmol (1 eq) of tricosan-12-amine in Synthetic Example 1 to obtain a ligand compound represented by Formula 1-6.

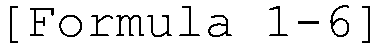

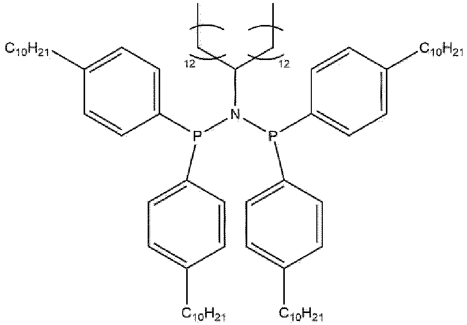

### N-(bis(4-decylphenyl)phosphaneyl)-1,1-bis(4-decylphenyl)-N-(heptacosan-14-yl)phosphanamine

¹H NMR (500 MHz, CDCl₃): δ 7.21(m, 8H), 7.18(m, 8H), 2.67(m, 8H), 2.47(m, 1H), 1.68(m, 8H), 1.48(m, 4H), 1.42(m, 4H), 1.29(m, 96H), 0.97(m, 18H)

### Synthetic Example 7: Synthesis of Ligand Compound Represented by Formula 1-7

The same method as Synthetic Example 1 was performed except that 20 mmol (2 eq) of 1-bromo-3-decylbenzene was used instead of 20 mmol (2 eq) of 1-bromo-4-(tripropylsilyl)benzene in Synthetic Example 1 to obtain a ligand compound represented by Formula 1-7.

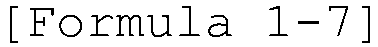

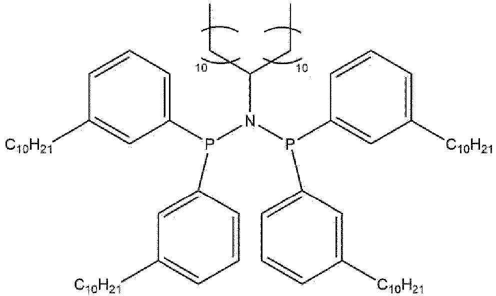

### N-(bis(3-decylphenyl)phosphaneyl)-1,1-bis(3-decylphenyl)-N-(tricosan-12-yl)phosphanamine

¹H NMR (500 MHz, CDCl₃): δ 7.39(m, 4H), 7.30(m, 4H), 7.23(m, 4H), 7.05(m, 4H), 2.68(m, 8H), 2.44(m, 1H), 1.62(m, 8H), 1.49(m, 4H), 1.34(m, 4H), 1.28(m, 88H), 0.97(m, 18H)

### Synthetic Example 8: Synthesis of Ligand Compound Represented by Formula 1-8

The same method as Synthetic Example 1 was performed except that 20 mmol (2 eq) of 1-bromo-3-decylbenzene was used instead of 20 mmol (2 eq) of 1-bromo-4-(tripropylsilyl)benzene, and 1 mmol (1 eq) of heptacosan-14-amine was used instead of 1 mmol (1 eq) of tricosan-12-amine in Synthetic Example 1 to obtain a ligand compound represented by Formula 1-8.

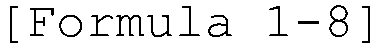

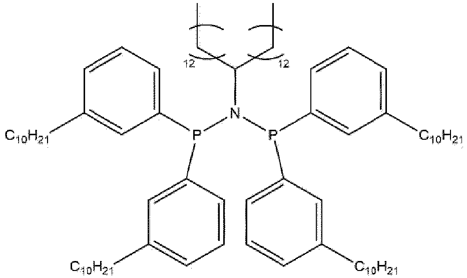

### N-(bis(3-decylphenyl)phosphaneyl)-1,1-bis(3-decylphenyl)-N-(heptacosan-14-yl)phosphanamine

¹H NMR (500 MHz, CDCl₃): δ 7.42(m, 4H), 7.35(m, 4H), 7.21(m, 4H), 7.07(m, 4H), 2.71(m, 8H), 2.50(m, 1H), 1.67(m, 8H), 1.47(m, 4H), 1.41(m, 4H), 1.35(m, 96H), 0.93(m, 18H)

### Comparative Synthetic Example 1: Synthesis of Ligand Compound Represented by Formula 14

20 mmol (2 eq) of 1-bromo-4-(tributylsilyl)benzene was dissolved in 20 ml of tetrahydrofuran, and then cooled to - 78°C. While maintaining the temperature, 20 mmol (2 eq) of n-butyllithium was added thereto dropwise, followed by stirring for 3 hours. Subsequently, 10 mmol (1 eq) of dichloro(diethylamino)phosphine dissolved in 10 ml of tetrahydrofuran was added thereto dropwise, followed by raising the temperature to room temperature and stirring overnight. Thereafter, the solvent was removed using vacuum. The first intermediate thus obtained without further purification was dissolved in 30 ml of n-hexane, and then HCl (in ether, 2 eq) was added, followed by stirring for 15 minutes and filtering. The filtrate was dried in vacuum. 2.1 mmol (2.1 eq) of the second intermediate thus obtained was dissolved in 3.8 ml of dichloromethane, and then 3 mmol (3 eq) of triethylamine was added. Then, 1 mmol (1 eq) of isopropylamine dissolved in dichloromethane was slowly added, and stirred at room temperature overnight. After removing the solvent in vacuum, the resultant was dissolved in 7.6 ml of n-hexane, and loaded onto the top of a silica of column chromatography. By using n-hexane containing 1 wt% of triethylamine, silica filtration was performed, and the solution thus obtained was concentrated to obtain a ligand compound represented by Formula 14.

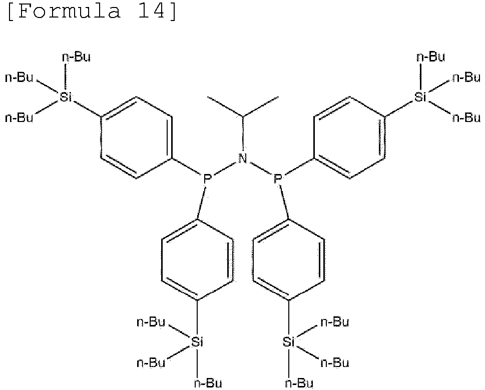

### N-(bis(4-(tributylsilyl)phenyl)phosphaneyl)-N-isopropyl-1,1-bis(4-(tributylsilyl)phenyl)phosphanamine

¹H NMR (500 MHz, C₆D₆): δ 8.12-7.22(br, 8H), 7.51(d, 8H), 3.87(m, 1H), 1.45-1.28(br, 48H), 1.25(d, 6H), 0.92(t, 36H), 0.92-0.79(br, 24H)

### Comparative Synthetic Example 2: Synthesis of Ligand Compound Represented by Formula 15

The same method as Comparative Synthetic Example 1 was performed except that 1 mmol (1 eq) of pentadecane-8-amine was used instead of 1 mmol (1 eq) of isopropylamine in Comparative Synthetic Example 1 to obtain a ligand compound represented by Formula 15.

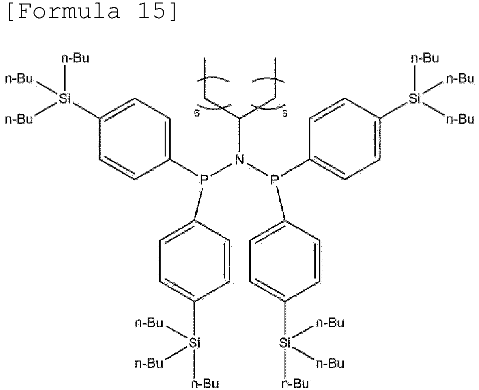

### N-(bis(4-(tributylsilyl)phenyl)phosphaneyl)-N-(pentadecane-8-yl)-1,1-bis(4-(tributylsilyl)phenyl)phosphanamine

¹H NMR (500 MHz, C₆D₆): δ 8.15-7.79(br, 4H), 7.52(s, 8H), 7.71-7.33(br, 4H), 3.59(m, 1H), 2.13(m, 2H), 1.84(m, 2H), 1.61-1.09(m, 92H), 1.09-0.10(m, 42H)

### Comparative Synthetic Example 3: Synthesis of Ligand Compound Represented by Formula 16

The same method as Comparative Synthetic Example 1 was performed except that 1 mmol (1 eq) of hentriacontane-16-amine was used instead of 1 mmol (1 eq) of isopropylamine in Comparative Synthetic Example 1 to obtain a ligand compound represented by Formula 16

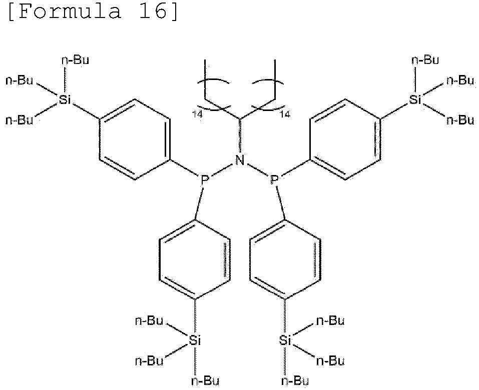

### N-(bis(4-(tributylsilyl)phenyl)phosphaneyl)-N-(hentriacontane-16-yl)-1,1-bis(4-(tributylsilyl)phenyl)phosphanamine

¹H NMR (500 MHz, C₆D₆): δ 8.14-7.78(br, 4H), 7.53(s, 8H), 7.70-7.34(br, 4H), 3.62(m, 1H), 2.11(m, 2H), 1.86(m, 2H), 1.62-1.10(br, 100H), 1.10-0.08(m, 66H)

### Comparative Synthetic Example 4: Synthesis of Ligand Compound Represented by Formula 17

The same method as Comparative Synthetic Example 1 was performed except that 1 mmol (1 eq) of pentatriacontane-18-amine was used instead of 1 mmol (1 eq) of isopropylamine in Comparative Synthetic Example 1 to obtain a ligand compound represented by Formula 17.

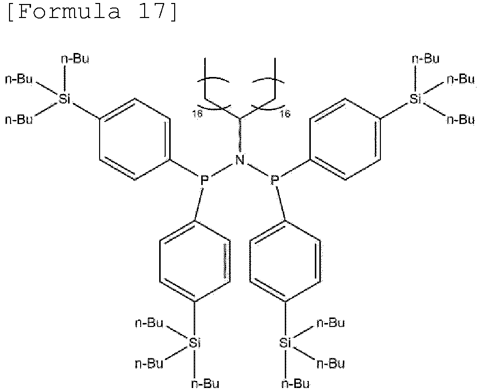

### N-(bis(4-(tributylsilyl)phenyl)phosphaneyl)-N-(pentatriacontane-18-yl)-1,1-bis(4-(tributylsilyl)phenyl)phosphanamine

¹H NMR (500 MHz, C₆D₆): δ 8.15-7.80(br, 4H), 7.53(s, 8H), 7.70-7.35(br, 4H), 3.63(m, 1H), 2.12(m, 2H), 1.85(m, 2H), 1.60-1.08(br, 108H), 1.08-0.07(m, 66H)

### Examples and Comparative Examples

### Example 1

Under an argon gas atmosphere, 0.5 mmol of chromium (III) chloride tetrahydrofuran (Cr(THF)₃Cl₃), 0.5 mmol of the ligand compound represented by Formula 1-1 according to Synthetic Example 1, and 0.5 mmol of N,N-dimethylanilinium tetrakis(pentafluorophenyl)borate (AB) as a cocatalyst were put in a flask, 30 ml of dichloromethane was added thereto, stirring was performed for 1 hour, and the solvent was removed in vacuum. After that, the resultant was dissolved in methylcyclohexane and filtered, and the solvent was removed in vacuum again, and the resultant was dissolved in methylcyclohexane to prepare a 0.15 mM (based on Cr) catalyst solution.

A Parr reactor with a volume of 600 ml was prepared, vacuum was applied at 120°C for 2 hours, the inside was replaced with argon, and the temperature was reduced to 70°C. Then, 180 ml of methylcyclohexane and 725 µmol of diisobutyl aluminum hydride as 2 ml of an activator were injected, and 5 ml of the catalyst solution (0.75 µmol Cr) was injected thereto. After stirring at 1,000 rpm for 2 minutes, the valve of an ethylene line adjusted to 40 bar was opened to fill the inside of the reactor with ethylene, and then, stirring was performed at 1,000 rpm for 60 minutes. The ethylene line valve was closed, the reactor was cooled to 0°C using a dry ice/acetone bath, unreacted ethylene was slowly ventilated, and 0.5 ml of nonane (GC internal standard) was injected. After stirring for 10 seconds, 2 ml of a liquid part of the reactor was taken and quenched with water, and an organic part thus obtained was filtered using a PTFE syringe filter to manufacture a GC-FID sample. Then, the distribution of a liquid product was analyzed by GC (Agilent Co., 6890N, Alltech AT-5 (30 mm x 0.32 mm ID x 0.25 µm; series no. 12446)). To a remaining solution, 400 ml of ethanol/HCl (10 vol% of aqueous 12 M HCl solution) was added, stirring and filtering were performed, and the amount of a solid product was analyzed. The polymer thus obtained was dried in a vacuum oven of 80°C overnight.

### Examples 2 to 8, and Comparative Examples 1 to 4

The same method as in Example 1-1 was performed except for changing the type of the catalyst as in Table 1 below.

**[Table 1]**

| Division | Ligand compound | Cocatalyst | Chromium source |
|---|---|---|---|
| Example 1 | Synthetic Example 1 (Formula 1-1) | AB | Cr(THF)₃Cl₃ |
| Example 2 | Synthetic Example 2 (Formula 1-2) | AB | Cr(THF)₃Cl₃ |
| Example 3 | Synthetic Example 3 (Formula 1-3) | AB | Cr(THF)₃Cl₃ |
| Example 4 | Synthetic Example 4 (Formula 1-4) | AB | Cr(THF)₃Cl₃ |
| Example 5 | Synthetic Example 5 (Formula 1-5) | AB | Cr(THF)₃Cl₃ |
| Example 6 | Synthetic Example 6 (Formula 1-6) | AB | Cr(THF)₃Cl₃ |
| Example 7 | Synthetic Example 7 (Formula 1-7) | AB | Cr(THF)₃Cl₃ |
| Example 8 | Synthetic Example 8 (Formula 1-8) | AB | Cr(THF)₃Cl₃ |
| Comparative Example 1 | Comparative Synthetic Example 1 (Formula 14) | AB | Cr(THF)₃Cl₃ |
| Comparative Example 2 | Comparative Synthetic Example 2 (Formula 15) | AB | Cr(THF)₃Cl₃ |
| Comparative Example 3 | Comparative Synthetic Example 3 (Formula 16) | AB | Cr(THF)₃Cl₃ |
| Comparative Example 4 | Comparative Synthetic Example 4 (Formula 17) | AB | Cr(THF)₃Cl₃ |

### Experimental Examples

The results of ethylene oligomerization reaction according to the Examples and Comparative Examples are shown in Table 2 below.

* Catalyst activity (ton/mol·Cr/hr): From the total weight (ton) value of the product obtained by combining the weights (ton) of the liquid product and solid product obtained, the catalyst activity was calculated.
* 1-C6, 1-C8, Iso-C6 and Iso-C8 selectivity (wt%): From the GC analysis results of the distribution of a liquid product, the contents of 1-hexene (1-C6), 1-octene (1-C8), the isomers thereof (Iso-C6 and Iso-C8) were calculated, and the wt% of 1-hexene, 1-octene and the isomers thereof based on the total weight of the product were calculated.
* Solid (wt%): The wt% of the solid product based on the total weight of the product was calculated. The solid means insoluble solids which are not dissolved in a solvent, and represents the degree of production of polyethylene with a carbon number of about 40 or more.

**[Table 2]**

| Division | Catalyst activity | 1-C6 and 1-C8 selectivity | | | Iso-C6 | Iso-C8 | Solid |
|---|---|---|---|---|---|---|---|
| | | 1-C6 | 1-C8 | Total | | | |
| | (ton/mol ·Cr/hr) | (wt%) | (wt%) | (wt%) | (wt%) | (wt%) | (wt%) |
| Example 1 | 260 | 36.12 | 48.35 | 84.47 | 3.31 | 0.28 | 0.58 |
| Example 2 | 266 | 37.15 | 47.31 | 84.46 | 3.28 | 0.33 | 0.45 |
| Example 3 | 240 | 36.32 | 46.72 | 83.04 | 3.32 | 0.28 | 0.67 |
| Example 4 | 246 | 38.55 | 46.53 | 85.08 | 3.35 | 0.33 | 0.63 |
| Example 5 | 225 | 36.31 | 48.85 | 85.16 | 3.42 | 0.26 | 0.35 |
| Example 6 | 230 | 37.63 | 47.06 | 84.69 | 3.34 | 0.33 | 0.41 |
| Example 7 | 209 | 36.52 | 46.44 | 82.96 | 3.30 | 0.30 | 0.44 |
| Example 8 | 214 | 38.73 | 47.22 | 85.95 | 3.29 | 0.34 | 0.32 |
| Comparative Example 1 | 176 | 28.33 | 54.58 | 82.91 | 3.78 | 0.39 | 0.15 |
| Comparative Example 2 | 185 | 30.23 | 49.56 | 79.79 | 3.56 | 0.36 | 0.35 |
| Comparative Example 3 | 171 | 39.25 | 39.02 | 78.27 | 3.15 | 0.33 | 0.67 |
| Comparative Example 4 | 139 | 40.62 | 38.42 | 79.04 | 3.11 | 0.42 | 0.23 |

As shown in Table 2 above, if an ethylene oligomerization reaction is performed using the catalyst composition including the ligand compound according to the present invention, it was confirmed that the most appropriate steric strain is formed from a ligand compound in which an alkyl group of 21 to 30 carbon atoms is substituted on the nitrogen atom of a diphosphino aminyl moiety, thereby reducing the production ratio of the isomers of 1-hexene and/or 1-octene without reducing the activity of the catalyst, and improving the catalytic activity, selectivity, and stability.

From the results, it was confirmed that if ethylene oligomerization is performed using the organochromium compound including the ligand compound and the catalyst composition of the present invention, a linear alpha-olefin may be prepared with excellent productivity due to high catalyst activity, and high selectivity to 1-hexene and 1-octene.

## Claims

1. A ligand compound represented by the following Formula 1: 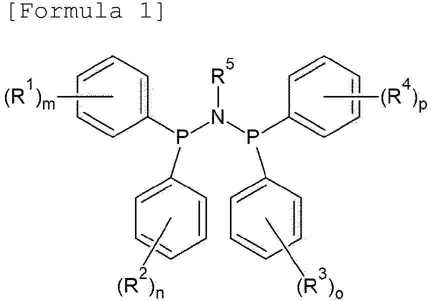 in Formula 1,
R¹ to R⁴ are each independently an alkyl group of 1 to 20 carbon atoms, a cycloalkyl group of 5 to 20 carbon atoms, an alkoxy group of 1 to 20 carbon atoms, or -Si(R⁷)₃,
m, n, o, and p are each independently 0 or an integer selected from 1 to 5, where if m, n, o, and p are 2 or more, a plurality of R¹ to R⁴ are the same or different from each other,
R⁵ is an alkyl group of 21 to 30 carbon atoms, and
R⁷ is an alkyl group of 1 to 20 carbon atoms, where a plurality of R⁷ are the same or different from each other.

2. The ligand compound according to claim 1, wherein R¹ to R⁴ are each independently an alkyl group of 1 to 20 carbon atoms, or -Si(R⁷)₃.

3. The ligand compound according to claim 1, wherein R¹ to R⁴ are each independently -Si(R⁷)₃, and R⁷ is an alkyl group of 1 to 10 carbon atoms, where a plurality of R⁷ are the same or different from each other.

4. The ligand compound according to claim 1, wherein R¹ to R⁴ are each independently a trimethylsilyl group, a triethylsilyl group, a tri-n-propylsilyl group, a tri-n-butylsilyl group, a tri-n-octylsilyl group or a dimethyl(n-octyl)silyl group.

5. The ligand compound according to claim 1, wherein m, n, o and p are 1.

6. The ligand compound according to claim 1, wherein R⁵ is a branched alkyl group of 21 to 30 carbon atoms.

7. The ligand compound according to claim 1, wherein the ligand compound represented by Formula 1 is represented by the following Formula 2 or Formula 3: 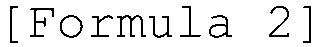 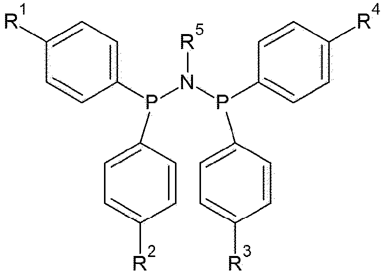 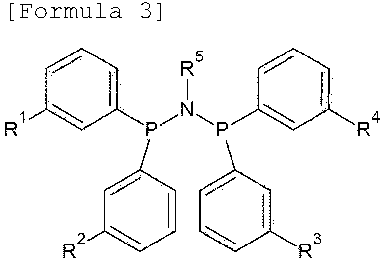 in Formula 2 and Formula 3, R¹ to R⁵ are each the same as defined in claim 1.

8. The ligand compound according to claim 1, wherein the ligand compound represented by Formula 1 is represented by the following Formula 4: 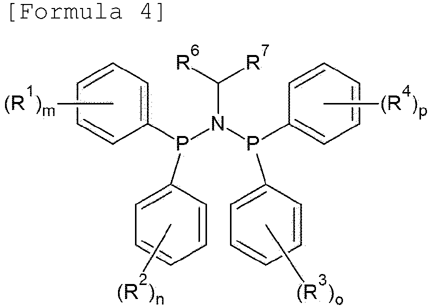
in Formula 4, R¹ to R⁴, m, n, o, and p are the same as defined in claim 1, and
R⁶ and R⁷ are each independently an alkyl group of 1 to 27 carbon atoms, where the sum of carbon atoms of R⁶ and R⁷ is 20 to 29.

9. The ligand compound according to claim 1, wherein the ligand compound represented by Formula 1 is represented by the following Formula 5 or Formula 6: 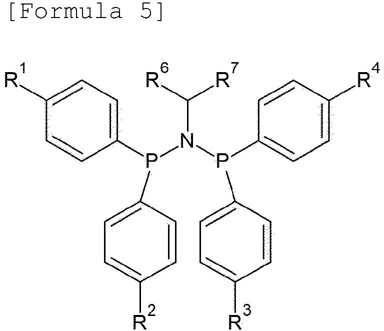 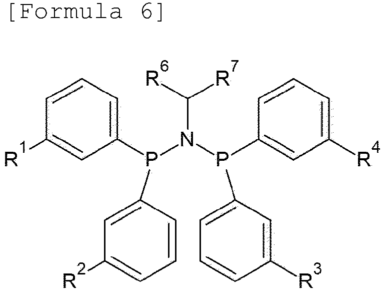
in Formulas 5 and 6, R¹ to R⁴ are the same as defined in claim 1, and
R⁶ and R⁷ are each independently an alkyl group of 1 to 27 carbon atoms, where the sum of carbon atoms of R⁶ and R⁷ is 20 to 29.

10. The ligand compound according to claim 1, wherein the ligand compound represented by Formula 1 is represented by any one among the following Formula 1-1 to Formula 1-12:

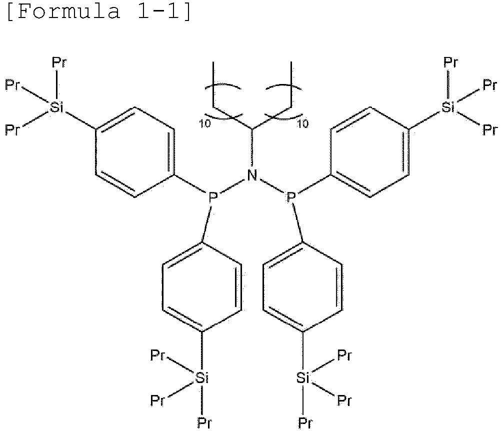

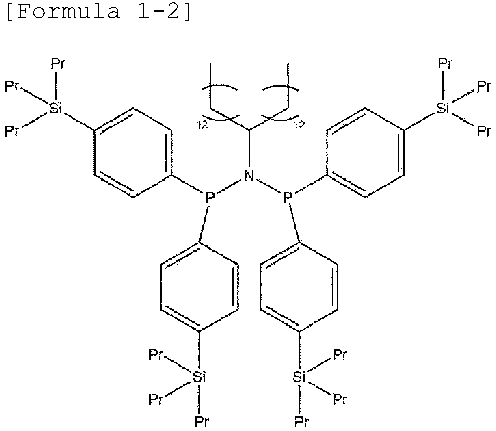

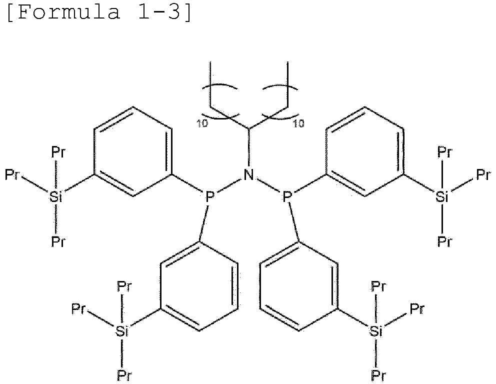

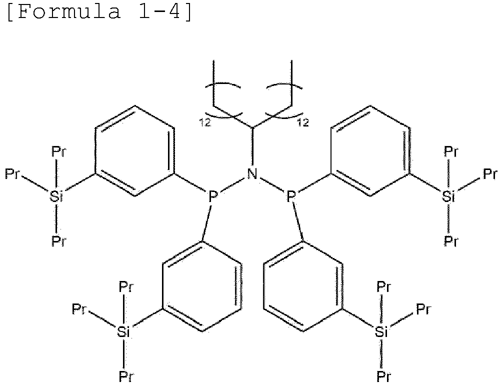

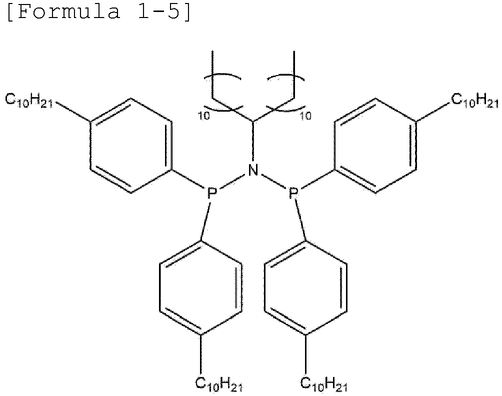

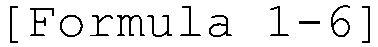

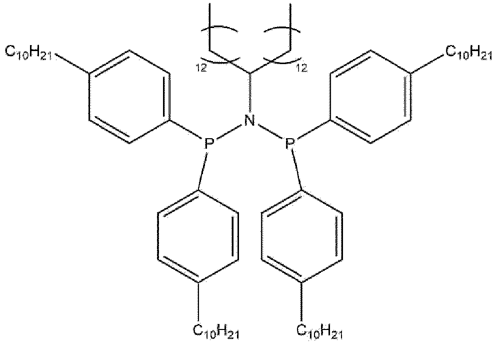

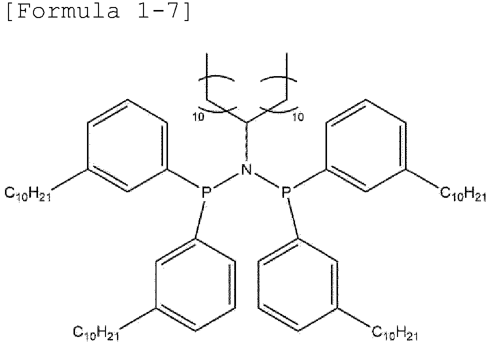

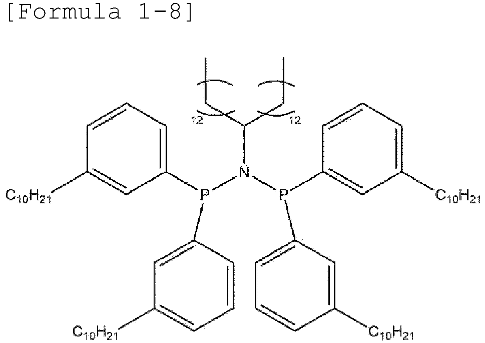

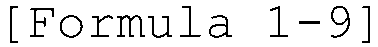

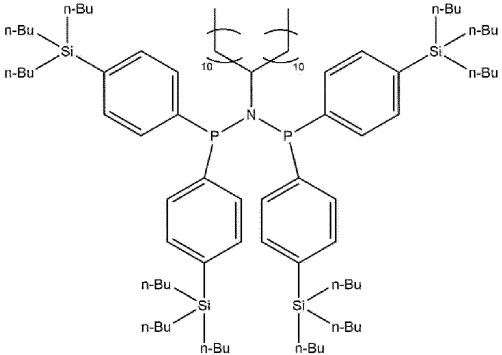

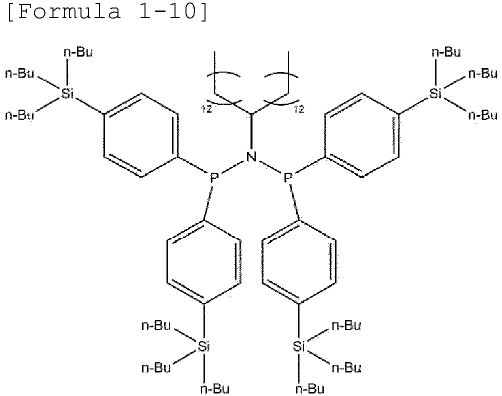

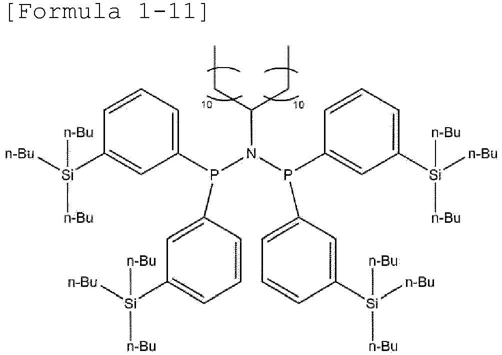

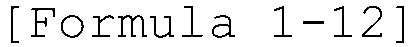

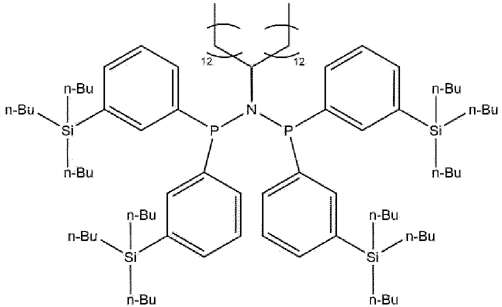

11. An organochromium compound comprising the ligand compound according to claim 1 and chromium making a coordination bond with the ligand compound.

12. The organochromium compound according to claim 11, wherein unshared electron pairs of one or more among N and two P in the ligand compound represented by Formula 1 make coordination bonds with the chromium.

13. A catalyst composition comprising the ligand compound according to claim 1, chromium and a cocatalyst.

14. The catalyst composition according to claim 13, wherein the chromium is derived from a chromium source, and the chromium source comprises one or more selected from the group consisting of chromium(III) acetylacetonate, chromium(III) chloride tetrahydrofuran, chromium(III) 2-ethylhexanoate, chromium(III) acetate, chromium(III) butyrate, chromium(III) pentanoate, chromium(III) laurate, chromium(III) tris(2,2,6,6-tetramethyl-3,5-heptanedionate) and chromium(III) stearate.

15. The catalyst composition according to claim 13, wherein the cocatalyst is one or more selected from the group consisting of the compounds represented by the following Formula 10 to Formula 13:
[Formula 10] -[Al(R¹³)-O]ₐ-
in Formula 10,
each R¹³ is independently a halogen group, a hydrocarbyl group of 1 to 20 carbon atoms, or a hydrocarbyl group of 1 to 20 carbon atoms, which is substituted with a halogen group, and
a is an integer of 2 or more,
[Formula 11] E(R¹⁴)₃
in Formula 11,
E is aluminum or boron, and
each R¹⁴ is independently hydrogen, a halogen group, a hydrocarbyl group of 1 to 20 carbon atoms, or a hydrocarbyl group of 1 to 20 carbon atoms, which is substituted with a halogen group,
[Formula 12] [L-H]⁺[G(Y)₄]⁻
[Formula 13] [L]⁺[G(Y)₄]⁻
in Formulas 12 and 13,
L is a neutral or cationic Lewis acid,
[L-H]⁺ is a brønsted acid,
G is an element in group 13, and
each Y is independently a substituted or unsubstituted alkyl group of 1 to 20 carbon atoms or a substituted or unsubstituted aryl group of 6 to 20 carbon atoms, where if the alkyl group or the aryl group is substituted, the substituent is a halogen group, a hydrocarbyl group of 1 to 20 carbon atoms, an alkoxy group of 1 to 20 carbon atoms, or an aryloxy group of 6 to 20 carbon atoms.

16. A method for preparing a linear alpha-olefin, the method comprising a step (S10) of oligomerizing ethylene in the presence of the catalyst composition according to claim 13.

17. The method for preparing a linear alpha-olefin according to claim 16, wherein the linear alpha-olefin is 1-hexene, 1-octene or a mixture of them.
